# EUROPÄISCHE PATENTSCHRIFT

(11) **EP 0 884 409 B1**
(45) Veröffentlichungstag und Bekanntmachung des Hinweises auf die Patenterteilung: **19.03.2003**
(21) Anmeldenummer: 98109997.1
(22) Anmeldetag: 02.06.1998
(51) Int. Cl.: D01H 13/22, D01G 31/00, D01H 13/14

(54) **Vorrichtung zur Erfassung von Parametern eines langgestreckten Prüfguts**
Device for detection of parameters of an elongated sample
Dispositif pour la détection d'un étalon allongé

(30) Priorität: 11.06.1997 CH 141097
(43) Veröffentlichungstag der Anmeldung: 16.12.1998
(73) Patentinhaber: ZELLWEGER LUWA AG, 8610 Uster (CH)
(72) Erfinder: Pirani, Peter, 8624 Grüt/Gossau ZH (CH)
(74) Vertreter: Ellenberger, Maurice

(56) Entgegenhaltungen:
- EP-A- 0 553 445
- WO-A-93/19359
- DE-A- 19 518 785
- MULLER J R: "FREMDFASERN IN DER SPINNEREI" MELLIAND TEXTILBERICHTE, INTERNATIONAL TEXTILE REPORTS, Bd. 76, Nr. 3, 1. März 1995, Seite 124/125, 128 XP000493651

## Beschreibung

Die Erfindung betrifft eine Vorrichtung zur Erfassung von Parametern eines langgestreckten Prüfguts, mit Elementen zur Erzeugung eines Strahlenganges in einem Bereiche des Prüfguts.

Aus der EP 0 401 600 ist bereits eine solche Vorrichtung für ein faden- oder drahtförmiges Prüfgut bekannt, bei der ein Messspalt vorgesehen ist, an dessen Seitenwänden je eine, Teil eines kapazitiven Messorgans bildende Messelektrode vorgesehen ist. Zusätzlich zum kapazitiven ist ein optisches Messorgan vorgesehen, mit einer an der einen Seite des Messspaltes angeordneten Lichtquelle und mit einem an der anderen Seite des Messspaltes angeordneten fotoelektrischen Element. Zur Erzeugung eines homogenen Beleuchtungsfeldes im Messspalt ist zwischen der Lichtquelle und dem Messspalt eine Blende oder ein kegelstumpfförmiger Lichtleiter vorgesehen.

Ein Nachteil dieser bekannten Vorrichtung ist darin zu sehen, dass neben dem Messspalt, für die Lichtquelle und für Mittel zur Strahlführung des Lichts, viel Raum beansprucht wird. Zudem müssen diese Mittel und die Lichtquelle eventuell lösbar aneinander oder auf einem Stützgestell befestigt sein, was insgesamt eine wenig raumsparende und wenig preisgünstige Lösung ergibt.

Aus der WO 93/19359 ist ein Verfahren und eine Vorrichtung zur Detektion von Verunreinigungen in einem textilen Prüfgut bekannt, womit die Reflexion am Prüfgut und zusätzlich der Durchmesser des Prüfguts erfasst wird. Dazu braucht es mindestens zwei Strahlungsquellen und einen oder mehrere Empfänger. Strahlungsquellen und Empfänger brauchen dabei genügenden Abstand zum Prüfgut wenn eine grossflächige, diffuse Beleuchtung des Prüfguts erreicht werden soll.

Aus der EP 0 553 445 ist ein Vorrichtung zur Detektion von Fremdfasem in einem langgestreckten textilen Gebilde bekannt, bei der das Gebilde in einem Messfeld geführt ist, das als ebener Schlitz in einem optisch transparenten Körper ausgebildet ist. Im transparenten Körper sind mehrere Lichtquellen und ein Detektor sowie verspiegelte Räume angeordnet, die eine diffuse Beleuchtung im Messfeld erzeugen sollen.

Aus der Schrift "Fremdfasern in der Spinnerei", Meilland Textilberichte 3, 1995, S. 124 ff, ist eine Anordnung für die Fremdfasererkennung im Garn bekannt, bei der neben einem Empfänger mehrere Optiken vorgesehen sind.

Die Erfindung, wie sie in den Patentansprüchen gekennzeichnet ist, löst nun die Aufgabe, eine Vorrichtung der genannten Art zu schaffen, die wenig Raum beansprucht, leicht herstellbar und kostengünstig ist.

Dies wird dadurch erreicht, dass mindestens eines der Elemente, die einen Strahlengang im Bereiche des Prüfguts erzeugen, flächenhaft ausgebildet ist. Ein solches Element ist ein flächenhaft ausgebildeter, diffuser Strahler, der als Lichtquelle wirkt. Im Bereiche der Elemente sollen femer Elektroden vorgesehen sein, die für die Strahlen des Strahlengangs durchlässig, also insbesondere im Falle von Lichtstrahlen durchsichtig sind und vorzugsweise als Schicht auf dem Element aufgebracht sind. Dem Element, das als Strahlen- oder Lichtquelle wirkt, ist ein Element zugeordnet, das als Detektor wirkt und Strahlen vom Strahler empfängt, wobei diese durch das Prüfgut abgeschattet oder am Prüfgut reflektiert sein können.

Die durch die Erfindung erreichten Vorteile sind insbesondere darin zu sehen, dass gleichzeitig optisch und kapazitiv arbeitende Sensoren sehr einfach, billig und raumsparend ausgeführt werden können. Damit kann beispielsweise gleichzeitig die Masse und der Querschnitt oder Durchmesser des Prüfguts erfasst werden. Der geringe Raumbedarf erlaubt es, in einem vorgegebenen Raum zusätzlich weitere Sensoren vorzusehen, die verschiedene Eigenschaften an einem Prüfgut erfassen. Beispielsweise können so neben Sensoren zur Erfassung der Ungleichmässigkeit von Masse und Durchmesser des Prüfguts weitere Sensoren zur Erfassung von Fremdstoffen oder der Oberflächenstruktur des Prüfguts vorgesehen werden. Oder, dieselbe Eigenschaft kann von zwei oder mehreren Sensoren erfasst werden, die verschiedene Charakteristiken aufweisen z. B. verschiedene spektrale Empfindlichkeiten. Wird die erfindungsgemässe Vorrichtung für textile Garne gebaut, so kann sie Teil eines Gamreinigers sein, der reduzierte Aussenmasse aufweist und somit leicht an ausgewählten Stellen in einer Textilmaschine eingebaut werden kann, was bekanntlich nicht immer möglich ist, da meist wenig Raum für Zusatzgeräte an einer Textilmaschine vorhanden ist, oder zumindest nicht dort wo die Erfassung des Garns für die Überwachung oder Messung des Garns sinnvoll ist.

Im folgenden wir die Erfindung anhand eines Beispiels und mit Bezug auf die beiliegenden Zeichnungen näher erläutert. Es zeigen
Figur 1 und 2 je eine vereinfachte Darstellung einer erfindungsgemässen Vorrichtung mit einem Prüfgut,
Figur 3 und 4 ein Teil eines Elementes der Vorrichtung und
Figur 5 eine Vorrichtung mit einem Messspalt.

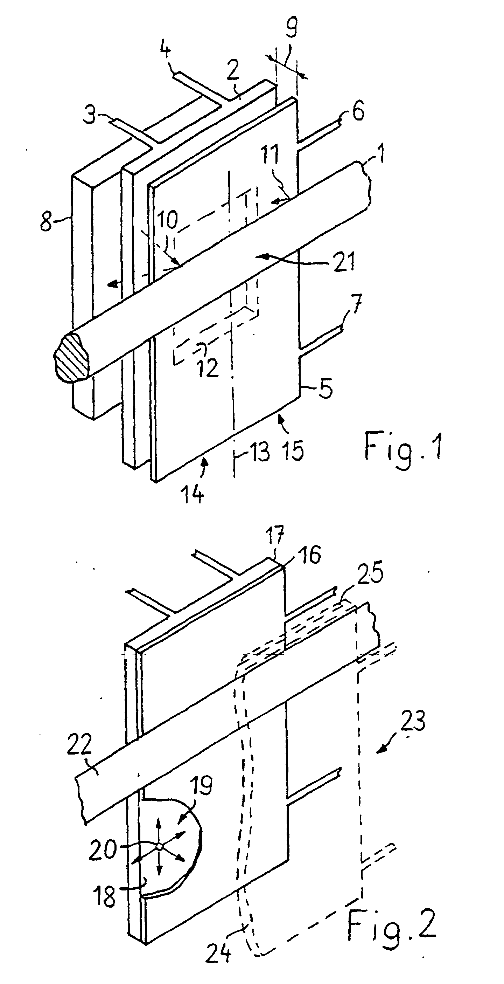

Fig. 1 zeigt eine erfindungsgemässe Vorrichtung für ein Prüfgut 1, die aus einem flächenhaft ausgebildeten Element 2, hier ein Strahler oder eine Strahlenquelle, mit elektrischen Anschlüssen 3 und 4, einer Elektrode 5, mit Anschlüssen 6 und 7 und aus einem weiteren hier ebenfalls flächenhaft ausgebildeten Element 8, einer Strahlensenke (meistens ein Detektor), mit hier nicht sichtbaren Anschlüssen, besteht. Bei dieser Ausführung ist die Elektrode 5 in einem Abstand 9 zum Strahler 2 angeordnet und beide erfassen einen Bereich 21 des Prüfguts 1.

Die Elektrode 5 ist für die Strahlung des Elementes 2 durchlässig, so dass ein Strahlengang 10, 11 zwischen den Elementen 2, 8, welcher vom Strahler 2 ausgeht und am Prüfgut 1 reflektiert wird, auf den Strahler 2 zurückfällt und, wenn auch der Strahler 2 durchlässig ist, durch den Strahler 2 hindurch auf den Detektor 8 fallen kann, wo eintreffende Strahlen beispielsweise in an sich bekannter Weise in ein elektrisches Signal gewandelt werden. Die Strahlung 10, 11 kann im Falle eines undurchlässigen Strahlers 2 auch durch ein Fenster 12 im Strahler 2 durch diesen hindurchtreten und auf den Detektor 8 fallen.

Es ist aber auch möglich, wie nachfolgend noch gezeigt, auf der dem Strahler 2 abgewandten Seite des Prüfguts 1, einen Detektor vorzusehen, der die vom Prügut 1 nicht abgeschattete Strahlung empfängt. Es ist auch möglich, die Vorrichtung in zwei Hälften aufzuteilen, die sich links und rechts der strichpunktierten Linie 13 ausbreiten, so dass jede Hälfte 14, 15 eine Strahlung mit bestimmten, von der Strahlung in der anderen Hälfte abweichenden Eigenschaft ausstrahlt. Beispielsweise könnte jede Hälfte 14, 15 Licht verschiedener Wellenlänge aussenden. Ebenso wäre es möglich die Elektrode 5 statt vor, hinter dem Element 2 anzuordnen.

Fig. 2 zeigt eine Vorrichtung, bei der eine Elektrode 16 auf der strahlenden Fläche 18 eines Strahlers 17 angeordnet ist. Vorzugsweise kann die Elektrode 16 als Schicht ausgebildet und auf der strahlenden Fläche 18 befestigt sein. Die strahlende Fläche 18 des Strahlers 17 strahlt seine Strahlen vorzugsweise diffus und in Richtungen ab wie sie für ein Flächenelement 20 durch Pfeile 19 dargestellt sind. Dazu gehören auch alle zwischen den einzelnen Pfeilen liegenden Richtungen. Man erkennt auch, dass das Prüfgut 22 hier beispielsweise bandförmig ausgebildet ist. Dem Element mit dem Strahler 16 und der Elektrode 17 gegenüberliegend ist ein Element 23 angeordnet, das als Detektor für den Empfang von Strahlen ausgebildet ist, wobei der eigentliche Detektor für die Strahlen mit dem Bezugszeichen 24 versehen und flächenhaft und mit einer Elektrode 25 versehen ausgebildet ist.

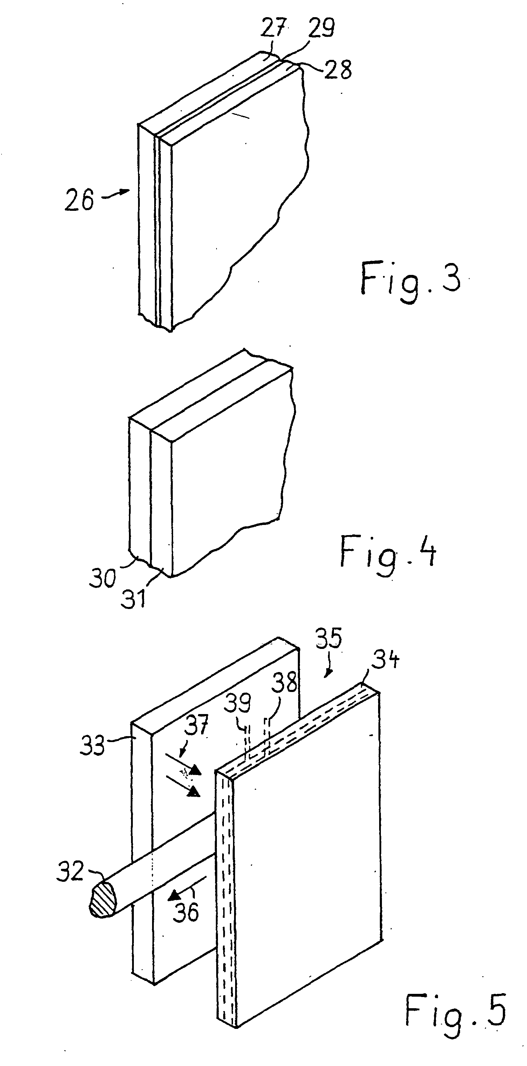

Fig. 3 zeigt einen Teil einer Vorrichtung bei der ein Element 26 aus mehreren Schichten 27, 28 aufgebaut ist. Dabei kann eine Elektrode 29 ebenfalls als Schicht ausgebildet, zwischen den Schichten 27, 28 angeordnet sein. Dies setzt natürlich voraus, dass mindestens die eine Schicht für Strahlen auch transparent ist. Es kann auch sein, dass die Elektrode 29 eine der für die Bildung der Strahlen notwendigen Schichten ist und damit zwei Funktionen ausübt. Dieser Aufbau eignet sich für Strahlenquellen wie für Strahlensenken (Detektoren) gleichermassen.

Fig. 4 zeigt einen weiteren Teil einer Vorrichtung bei der mehrere Elemente hinter- oder übereinander angeordnet sind. Man erkennt hier beispielsweise zwei Strahler 30 und 31 von denen jeder Strahlen mit bestimmten Eigenschaften aussendet. Solche Eigenschaften sind beispielsweise verschiedene Wellenlängen der Strahlung, verschiedene Frequenzen oder Modulationen usw.

Fig. 5 zeigt eine Vorrichtung für ein Prüfgut 32 mit einer Strahlenquelle 33 und einer Strahlensenke 34, die so zueinander stehen, dass ein Messspalt 35 für das Prüfgut 32 entsteht, das vorzugsweise in seiner Längsrichtung bewegt wird, so dass eine Relativbewegung entsprechend einem Pfeil 36 zwischen der Vorrichtung oder dem Messspalt 35 und dem Prüfgut 32 entsteht.

Die Wirkungsweise der erfindungsgemässen Vorrichtung ist wie folgt:
Zur Erfassung von Parametern an einem langgestreckten Prüfgut 1 wird dieses in an sich bekannter und deshalb hier nicht näher dargestellten Weise an einem Sensor, wie er durch Elemente mit Bezugszeichen 2, 5, 8, 16, 17 usw. dargestellt ist, vorbeibewegt. Mit dieser Vorrichtung soll beispielsweise kapazitiv die Masse und optisch der Durchmesser oder Querschnitt des Prüfguts bzw. deren Ungleichmässigkeiten über die Länge erfasst werden. Anstelle oder zusätzlich zu den Ungleichmässigkeiten können aber auch z.B. bei Garn die Haarigkeit, der Fremdfasergehalt usw. erfasst werden. Die Erfassung kann zum Zwecke einer Messung oder der Überwachung vorgenommen werden. Für den nachfolgenden Teil der Beschreibung sei angenommen, dass dieser Parameter einerseits durch ein elektrisches Feld und andererseits durch eine Strahlung hier in Form von Lichtstrahlen erfasst werden soll.

Für eine erste Erfassung wird deshalb das Prüfgut 1 in seinem Bereiche 21 durch Lichtstrahlen vom Strahler 2 beleuchtet, die durch eine durchsichtige Elektrode 5 weitgehend ungedämpft hindurchtreten können. Die Lichtstrahlen werden möglichst diffus abgegeben wie dies aus der Fig. 2 ersichtlich ist. Diese können im Sinne einer Durchlichtmessung von einem Empfänger oder Strahlensenke 34 (Fig. 5) erfasst werden. Dann erhält der Empfänger 34, bedingt durch Absorption, Reflexion und Streuung, nur einen Teil der vom Strahler 2 ausgesendeten Strahlung. Aus der empfangenen Lichtmenge kann so in bekannter Weise ein Signal abgeleitet werden, das den Verlauf des gesuchten Parameters darstellt.

Für eine zweite Erfassung kann derselbe Bereich 21 des Prüfguts 1, 32 durch ein elektrisches Feld 37 (Fig. 5) hindurchbewegt werden, das zwischen Elektroden liegt, wie sie im Sender 33 und Empfänger 34 vorhanden und, wie in Fig. 1 bis 3 dargestellt, ausgebildet sind. Zwischen den Elektroden, die im Empfänger 34 vorzugsweise einen gleichen Aufbau haben wie im Sender 31, lassen sich die durch das Prüfgut verursachten Veränderungen des elektrischen Feldes in bekannter Weise messen, so dass auch so ein Verlauf des gesuchten Parameters erhalten werden kann.

Sind die Elektroden nicht durchsichtig ausgebildet, so können die Lichtstrahlen durch ein Fenster 12 hindurchtreten und so das Prüfgut 1 beaufschlagen. Reflektierte Lichtstrahlen können ebenso durch das Fenster 12 und den Sender hindurch auf den Detektor 8 gelangen, während transmittierte Lichtstrahlen durch ein entsprechendes Fenster in der Elektrode des Empfängers 34 auf einen Detektor gelangen können, wo deren verbleibende Intensität erfasst wird. Bei stark diffuser Strahlung ist immer genügend Strahlung vorhanden, die durch das Prüfgut beeinflusst wird, und die durch die Fenster hindurchtritt.

Verwendet man mehrere Farben für eine Durchlicht- und/oder Auflichterfassung, so kann auch eine entsprechende Anzahl Detektoren eingesetzt werden, die jeweils nur in einer Farbe selektiv sind. Eine Farbselektion kann zudem auch durch Abgriffe 38, 39 für Elektronen in verschiedener Tiefe eines einzigen Detektors erfolgen.

Alle dargestellten Sender und Empfänger mit Schichten können zudem eine transparente Schutzschicht gegen schädliche Einflüsse der Umgebung wie Feuchtigkeit, Sauerstoff, Abrasion usw. aufweisen.

Als flächenhaft ausgebildete Strahler eignen sich beispielsweise leuchtende Polymere wie z.B. diejenigen der Firma Cambridge Display Technology in Cambridge UK besonders gut .

Als flächenhafte Ausbildung verstehen wir die Ausbildung eines Elementes derart, dass die Strahlen aussendende Fläche eine Länge und Breite aufweist, die wesentlich grösser ist als die Tiefe des Elementes, welche Tiefe sich etwa senkrecht zu der genannten Fläche erstreckt. Das strahlenerzeugende Element ist mit anderen Worten dadurch gekennzeichnet, dass es keine weiteren optischen Elemente aufweist, die die Strahlen formen, umlenken, streuen usw. Damit ergibt sich eine platzsparende Anordnung. Die Fläche, welche Strahlen aussendet, ist so gross wie das Messfeld. So liegen auch Anschlüsse 3,4 und 6, 7 (Fig. 1) zum Zuführen von elektrischer Energie nahe beim Prüfgut, vorzugsweise nur durch den Abstand des Prüfguts von der strahlenden Fläche und einen Teil der Tiefe des Elementes getrennt.

## Patentansprüche

1. Vorrichtung zur Erfassung von Parametern eines langgestreckten Prüfguts (1), mit Elementen (2, 8) zur Erzeugung eines Strahlenganges (10) in einem Bereich (21) des Prüfguts, **gekennzeichnet durch** ein flächenhaft und eben ausgebildetes Element (2), dessen Länge und Breite seine Tiefe wesentlich übersteigt und das eine strahlende Fläche (18), gebildet **durch** Länge und Breite des Elementes (2), zur Erzeugung eines Strahlenganges aufweist, die Licht-Strahlen diffus abstrahlt.

2. Vorrichtung nach Anspruch 1, **dadurch gekennzeichnet, dass** das Element (2) eine Strahlen bildende Schicht aufweist.

3. Vorrichtung nach Anspruch 1, **dadurch gekennzeichnet, dass** die strahlende Fläche (18) der Grösse eines Messfeldes im Bereich (21) des Prüfguts (1) entspricht.

4. Vorrichtung nach Anspruch 1, **dadurch gekennzeichnet, dass** mindestens zwei Strahlengänge (10, 11) mit verschiedenen Wellenlängen vorgesehen sind um denselben Bereich (21) des Prüfguts zu erfassen, wobei dies entweder durch die Aufteilung (14, 15) der strahlenden Fläche (18) oder durch die Hintereinanderanordnung mehrerer strahler (30, 31) erfolgt.

5. Vorrichtung nach Anspruch 4, **dadurch gekennzeichnet, dass** die Strahlengänge zur Erfassung desselben Bereiches (21) des Prüfguts einander überlagert sind.

6. Vorrichtung nach Anspruch 1, **dadurch gekennzeichnet, dass** das Element eine für Strahlen transparente Schutzschicht aufweist.

7. Vorrichtung nach Anspruch 1, **dadurch gekennzeichnet, dass** dem Element zur Erzeugung des Strahlenganges ein ebenfalls flächenhaft ausgebildeter Detektor (8, 31) für den Empfang der Strahlen zugeordnet ist.

8. Vorrichtung nach Anspruch 1, **dadurch gekennzeichnet, dass** als Strahler ein leuchtendes Polymer (LEP) vorgesehen ist.

9. Vorrichtung nach Anspruch 1, **dadurch gekennzeichnet, dass** zwischen dem Element und dem Prüfgut eine Relativbewegung erfolgt.

## Claims

1. Device for registering parameters of an elongated test material (1), said device comprising elements (2, 8) for generating a beam path (10) in a region (21) of the test material, **characterised by** an element (2) of planar construction which is flat and which has a length and width substantially greater than the depth of the element and comprising a radiant face (18), formed by the length and width of the element (2) for generating a beam path which is designed for the emission of diffuse light.

2. Device according to Claim 1, **characterised in that** the element (2) comprises a layer for forming the beams.

3. Device according to Claim 1, **characterised in that** the radiant face (18) is the same size as the measuring field in the region (21) of the test material (1).

4. Device according to Claim 1, **characterised in that** at least two beam paths (10, 11) having different wavelenghts are provided in order to register the same region (21) of the test material, whereas the radiant face (18) is split into parts (14, 15) or whereas several radiant faces (30, 31) are arranged behind one another.

5. Device according to Claim 4, **characterised in that** the beam paths are superimposed on one another for registering the same region (21) of the test material.

6. Device according to Claim 1, **characterised in that** the element comprises a transparent protective layer.

7. Device according to Claim 1, **characterised in that** a detector (8, 31) for receiving the beams which is likewise of planar construction is assigned to the element for generating the beam path.

8. Device according to Claim 1, **characterised in that** a luminous polymer (LEP) is provided by way of radiator.

9. Device according to Claim 1, **characterised in that** a relative movement takes place between the element and the test material.

## Revendications

1. Dispositif pour la détection de paramètres d'un échantillon de contrôle allongé (1), avec des éléments (2, 8) pour produire une trajectoire de rayons (10) dans une zone (21) de l'échantillon de contrôle, **caractérisé par** un élément réalisé d'une manière plane et en forme de face (2) dont la longueur et largeur dépassent considérablement sa profondeur et qui présente une face de rayonnement (18), formée par la longueur et largeur de l'élément (2), pour produire une trajectoire de rayons qui fait rayonner d'une manière diffuse les rayons de lumière.

2. Dispositif selon la revendication 1, **caractérisé en ce que** l'élément (2) présente une couche formant des rayons.

3. Dispositif selon la revendication 1, **caractérisé en ce que** la face de rayonnement (18) correspond à la grandeur d'un champ de mesure dans la zone (21) de l'échantillon de contrôle (1).

4. Dispositif selon la revendication 1, **caractérisé en ce qu'**au moins deux trajectoires de rayons (10, 11) de différentes longueurs d'ondes sont prévues pour détecter la même zone (21) de l'échantillon de contrôle, où cela a lieu soit par la répartition (14, 15) de la face de rayonnement (18) soit par la disposition successive de plusieurs émetteurs de rayonnement (30, 31).

5. Dispositif selon la revendication 4, **caractérisé en ce que** les trajectoires de rayons, pour détecter la même zone (21) de l'échantillon de contrôle, sont superposées.

6. Dispositif selon la revendication 1, **caractérisé en ce que** l'élément présente une couche de protection transparente aux rayons.

7. Dispositif selon la revendication 1, **caractérisé en ce qu'**il est associé à l'élément pour produire la trajectoire de rayons un détecteur (8, 31) également réalisé en forme de face pour la réception des rayons.

8. Dispositif selon la revendication 1, **caractérisé en ce qu'**un polymère lumineux (LEP) est prévu comme émetteur de rayonnement.

9. Dispositif selon la revendication 1, **caractérisé en ce qu'**un déplacement relatif a lieu entre l'élément et l'échantillon de contrôle.
